# EUROPEAN PATENT APPLICATION

(11) **EP 4 218 924 A1**
(43) Date of publication of application: **02.08.2023**
(21) Application number: 23153717.6
(22) Date of filing: 27.01.2023
(51) Int. Cl.: A61N 5/06, A61B 1/04

(54) **LIGHT BEAM IRRADIATION PLANNING APPARATUS, LIGHT BEAM IRRADIATION SUPPORTING APPARATUS, MEDICAL INFORMATION PROCESSING SYSTEM, AND MEDICAL INFORMATION PROCESSING METHOD**

(30) Priority: 27.01.2022 JP 2022011080
(71) Applicant: Canon Medical Systems Corporation, Tochigi 324-0036 (JP)
(72) Inventor: ICHIHASHI, Masahide, Otawara-shi, 324-0036 (JP)
(74) Representative: Marks & Clerk LLP

(57) **Abstract**

A light beam irradiation planning apparatus (20) according to embodiments includes an identifying unit (23b) configured to identify a tumor site included in a medical image, an irradiation mode setting unit (23c) configured to set an irradiation mode to the tumor site, the irradiation mode satisfying a criterion for a chemical reaction of a drug binding to a tumor by a light beam, and a guidance information generating unit (23d) configured to generate guidance information for guiding light beam irradiation by a light beam irradiation device based on the set irradiation mode.

## Description

### FIELD

Embodiments described herein relate generally to a light beam irradiation planning apparatus, a light beam irradiation supporting apparatus, a medical information processing system, and a medical information processing method.

### BACKGROUND

Photoimmunotherapy (near-infrared light therapy) is known as one of treatments for tumors such as cancer. In the photoimmunotherapy, a drug that binds to tumors is first administered. More specifically, a drug that is obtained by combining antibodies binding to a substance specifically existing on a surface of a tumor with a substance that emits fluorescence by absorbing a light beam (a light-sensitive substance) is administered to a subject. Thereafter, a light beam irradiation device (diffuser) emits a light beam to the drug to cause a chemical reaction, thereby killing tumor cells. More specifically, the light-sensitive substance also binds to tumors via binding to the antibodies. The light beam emitted from the light beam irradiation device causes a chemical reaction in the light-sensitive substance, thereby killing the tumor cells.

In order to cause a chemical reaction with respect to an administered drug, it is necessary to emit a light beam to all of affected sites without exception. In the related art, users such as physicians have placed marks directly on an area to be irradiated to perform light beam irradiation within the range of their visual discretion. However, with this method in the related art, certain positions may not be irradiated with a light beam, or may be insufficiently irradiated with a light beam or excessively irradiated with a light beam.

### Summary of Invention

A light beam irradiation planning apparatus including: an identifying unit configured to identify a tumor site included in a medical image; an irradiation mode setting unit configured to set an irradiation mode to the tumor site, the irradiation mode satisfying a criterion for a chemical reaction of a drug binding to a tumor by a light beam; and a guidance information generating unit configured to generate guidance information for guiding light beam irradiation by a light beam irradiation device based on the set irradiation mode.

The irradiation mode setting unit may set, as the irradiation mode, any of an irradiation dose, an irradiation time, and an irradiation intensity in a range including the tumor site.

The irradiation mode setting unit may set, as the irradiation mode, any of an irradiation dose, an irradiation time, and an irradiation intensity in an irradiation range group including a combination of a plurality of irradiation ranges.

The irradiation mode setting unit may set the irradiation range group so that a part of each irradiation range overlaps with other irradiation ranges adjacent to the irradiation range.

The irradiation mode setting unit may set the irradiation range group in which sizes of the irradiation ranges are substantially identical to each other.

The irradiation mode setting unit may set the irradiation mode based on anatomical information corresponding to at least one of the tumor site or a surrounding area of the tumor site, and the criterion.

The irradiation mode setting unit may output information for identifying whether the irradiation mode in the range including the tumor site satisfies the criterion, based on an irradiation condition of the light beam irradiation device and the anatomical information.

The guidance information generating unit may generate, as the guidance information, a procedure of light beam irradiation by the light beam irradiation device.

The guidance information generating unit may generate, as the guidance information, at least one of an irradiation range including the tumor site and an irradiation dose, an irradiation time, and an irradiation intensity in the irradiation range.

The irradiation mode setting unit may set, as the irradiation mode, an irradiation dose according to a depth from a body surface to the tumor site.

The irradiation mode may include an irradiation direction to the tumor site.

The irradiation mode may include whether a puncture is applied to a subject by the light beam irradiation device.

The guidance information generating unit may generate, as the guidance information, information indicating a position at a body surface of the subject, which is punctured by the light beam irradiation device.

A light beam irradiation supporting apparatus including: an acquisition unit configured to acquire a preset setting irradiation mode to a tumor site included in a medical image; an actual irradiation mode acquisition unit configured to acquire an actual irradiation mode indicating that the tumor site is actually irradiated; and a provision unit configured to provide irradiation supporting information for supporting light beam irradiation by a light beam irradiation device based on the setting irradiation mode and the actual irradiation mode.

The provision unit may provide, as the irradiation supporting information, information indicating the setting irradiation mode together with information indicating the actual irradiation mode.

The setting irradiation mode may include a distribution of an irradiation dose or an irradiation time set to a range including the tumor site, the actual irradiation mode may include a distribution of an irradiation dose or an irradiation time, which is obtained by the range being actually irradiated, and the irradiation supporting information may be information that enables identification of a relationship between the distribution of the irradiation dose or the irradiation time included in the setting irradiation mode and the distribution of the irradiation dose or the irradiation time included in the actual irradiation mode.

The actual irradiation mode acquisition unit may sequentially acquire the actual irradiation mode while light beam irradiation is performed by the light beam irradiation device, and the provision unit may provide the irradiation supporting information while sequentially updating the irradiation supporting information according to the actual irradiation mode.

The provision unit may provide, as the irradiation supporting information, a distribution of the relationship in the range.

The provision unit may provide, as the irradiation supporting information, a superimposed image formed by superimposing the distribution of the irradiation dose or the irradiation time included in the setting irradiation mode, or the distribution of the relationship on an optical image of the range that is sequentially acquired.

The actual irradiation mode acquisition unit may acquire the actual irradiation mode by performing color conversion of the optical image of the range that is sequentially acquired and identifying a position that is being irradiated with a light beam by the light beam irradiation device, in the range.

The setting irradiation mode may include a distribution of an irradiation dose or an irradiation time set to an irradiation range group including a combination of a plurality of irradiation ranges, and the provision unit may provide, as the irradiation supporting information, information that enables identification of an irradiation range with a difference between the irradiation dose or the irradiation time included in the setting irradiation mode and the irradiation dose or the irradiation time included in the actual irradiation mode among the irradiation ranges.

A medical information processing system including: an identifying unit configured to identify a tumor site included in a medical image; an irradiation mode setting unit configured to set an irradiation mode to the tumor site, the irradiation mode satisfying a criterion for a chemical reaction of a drug binding to a tumor by a light beam; a guidance information generating unit configured to generate guidance information for guiding light beam irradiation by a light beam irradiation device based on the set irradiation mode; and a provision unit configured to provide the guidance information.

A medical information processing system including: an identifying unit configured to identify a tumor site included in a medical image; an irradiation mode setting unit configured to set a setting irradiation mode to the tumor site, the setting irradiation mode satisfying a criterion for a chemical reaction of a drug binding to a tumor by a light beam; an acquisition unit configured to acquire the setting irradiation mode; an actual irradiation mode acquisition unit configured to acquire an actual irradiation mode indicating that the tumor site is actually irradiated; and a provision unit configured to provide irradiation supporting information for supporting light beam irradiation by a light beam irradiation device based on the setting irradiation mode and the actual irradiation mode.

A medical information processing method including: identifying a tumor site included in a medical image; setting an irradiation mode to the tumor site, the irradiation mode satisfying a criterion for a chemical reaction of a drug binding to a tumor by a light beam; and generating guidance information for guiding light beam irradiation by a light beam irradiation device based on the set irradiation mode.

A medical information processing method including: acquiring a preset setting irradiation mode to a tumor site included in a medical image; acquiring an actual irradiation mode indicating that the tumor site is actually irradiated; and providing irradiation supporting information for supporting light beam irradiation by a light beam irradiation device based on the setting irradiation mode and the actual irradiation mode.

### BRIEF DESCRIPTION OF THE DRAWINGS

FIG. 1 is a block diagram illustrating an example of a configuration of a medical information processing system according to a first embodiment;
FIG. 2 is a flowchart illustrating a flow of processing of a light beam irradiation planning apparatus according to the first embodiment;
FIG. 3 is a diagram illustrating an example of an irradiation region group according to the first embodiment;
FIG. 4 is a diagram illustrating an example of a light beam irradiation device according to the first embodiment;
FIG. 5 is a diagram illustrating an example of guidance information according to the first embodiment;
FIG. 6 is a diagram illustrating omission in irradiation;
FIG. 7 is a diagram illustrating an example of photoimmunotherapy according to the first embodiment;
FIG. 8 is a block diagram illustrating an example of a configuration of a medical information processing system according to a second embodiment;
FIG. 9 is a flowchart illustrating a flow of processing of a light beam irradiation supporting apparatus according to the second embodiment;
FIG. 10A is a diagram illustrating an example of irradiation supporting information according to the second embodiment;
FIG. 10B is a diagram illustrating an example of irradiation supporting information according to the second embodiment;
FIG. 10C is a diagram illustrating an example of irradiation supporting information according to the second embodiment; and
FIG. 10D is a diagram illustrating an example of irradiation supporting information according to the second embodiment.

### DETAILED DESCRIPTION

Embodiments are described in detail below with reference to the drawings.

### First Embodiment

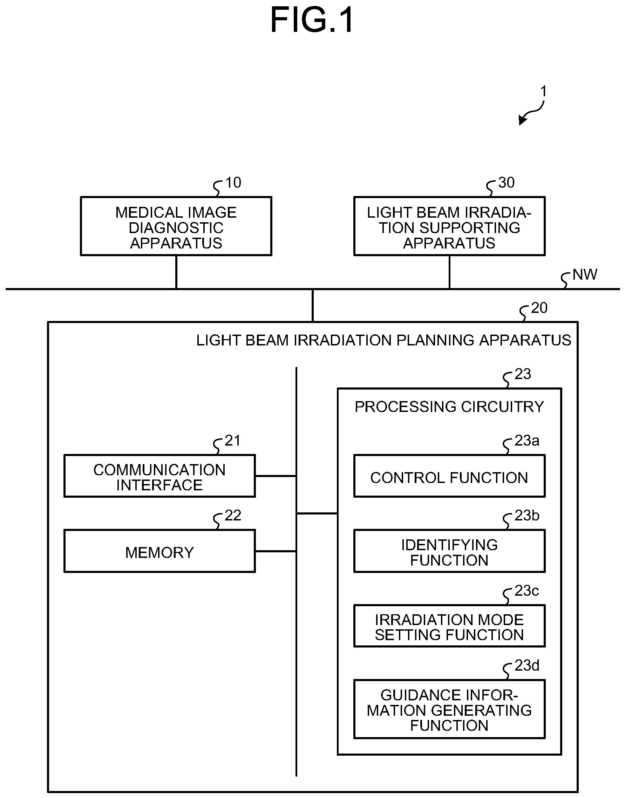

A medical information processing system 1 including a medical image diagnostic apparatus 10, a light beam irradiation planning apparatus 20, and a light beam irradiation supporting apparatus 30 will be described with reference to FIG. 1. The medical image diagnostic apparatus 10, the light beam irradiation planning apparatus 20, and the light beam irradiation supporting apparatus 30 are connected to each other via a network NW.

The medical image diagnostic apparatus 10 is an apparatus that acquires medical images from a subject. The medical image diagnostic apparatus 10 is, for example, an X-ray computed tomography (CT) apparatus, a magnetic resonance imaging (MRI) apparatus, or other apparatuses.

The light beam irradiation planning apparatus 20 uses the medical images acquired by the medical image diagnostic apparatus 10 to plan light beam irradiation in photoimmunotherapy. Specifically, the light beam irradiation planning apparatus 20 identifies a tumor site included in a medical image, sets an irradiation mode to the tumor site, which satisfies a criterion for a chemical reaction of a drug binding to a tumor by a light beam, and generates guidance information for guiding light beam irradiation by a light beam irradiation device based on the set irradiation mode. The provision of the guidance information to a user during the execution of photoimmunotherapy enables efficient light beam irradiation. For example, the light beam irradiation planning apparatus 20 is provided with a communication interface 21, a memory 22, and processing circuitry 23, as illustrated in FIG. 1.

The communication interface 21 is composed of, for example, a network card, a network adapter, or the like. The communication interface 21 transmits and receives various pieces of information to and from devices connected via the network NW under the control of the processing circuitry 23.

The memory 22 is composed of, for example, storage devices such as semiconductor memory devices such as random access memory (RAM), flash memory, and the like, hard disks, and optical disks. The memory 22 stores various pieces of information supplied by the processing circuitry 23. In addition, the memory 22 stores computer programs to be executed by the processing circuitry 23.

The processing circuitry 23 is composed of, for example, arithmetic processing devices such as central processing unit (CPU) and micro-processing unit (MPU). The processing circuitry 23 reads and executes computer programs stored in the memory 22 to function as a control function 23a, an identifying function 23b, an irradiation mode setting function 23c, and a guidance information generating function 23d. The identifying function 23b is an example of an identifying unit. The irradiation mode setting function 23c is an example of an irradiation mode setting unit. The guidance information generating function 23d is an example of a guidance information generating unit.

The control function 23a controls the communication interface 21 to control transmission and reception of data via the network NW. For example, the control function 23a receives a medical image from the medical image diagnostic apparatus 10. The control function 23a also transmits the guidance information generated by the guidance information generating function 23d to the light beam irradiation supporting apparatus 30.

The identifying function 23b identifies a tumor site T1 included in the medical image. The irradiation mode setting function 23c sets an irradiation mode for the identified tumor site T1, which satisfies a criterion for a chemical reaction of a drug binding to a tumor by a light beam. The guidance information generating function 23d generates guidance information for guiding light beam irradiation by the light beam irradiation device based on the set irradiation mode. The details of processing executed by the identifying function 23b, the irradiation mode setting function 23c, and the guidance information generating function 23d will be described later.

The light beam irradiation supporting apparatus 30 provides guidance information transmitted from the light beam irradiation planning apparatus 20 to a user such as a physician. For example, the light beam irradiation supporting apparatus 30 is provided with a display, and allows the guidance information to be displayed on the display.

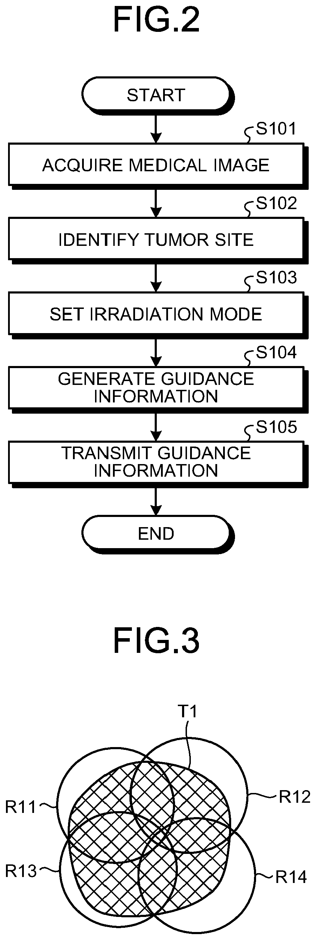

Under such a configuration, the light beam irradiation planning apparatus 20 in the medical information processing system 1 enables efficient light beam irradiation in the photoimmunotherapy. The details of processing executed by the light beam irradiation planning apparatus 20 will be described below with a flowchart illustrated in FIG. 2.

First, the control function 23a acquires a medical image (step S101). For example, the medical image of a subject is imaged by the medical image diagnostic apparatus 10 during diagnosis of the subject. The control function 23a acquires the medical image from the medical image diagnostic apparatus 10 or an image storage device that stores the medical image. An example of such an image storage device includes a picture archiving and communication system (PACS).

Next, the identifying function 23b identifies the tumor site T1 included in the medical image (step S102). For example, the identifying function 23b can identify the tumor site T1 by contour extraction. A method for identifying the tumor site T1 is not limited, and for example, Otsu's binarization method based on pixel values, the region expansion method, the snake method, the graph cut method, the mean-shift method, and other methods can be used to identify the tumor site T1. The identifying function 23b can also identify the tumor site T1 by using a learned model that has been machine-learned to identify the tumor site T1 from the medical image. In a case in which the medical image is a three-dimensional image, such as an MRI image or an X-ray CT image, the identifying function 23b can identify a three-dimensional region as the tumor site T1.

Next, the irradiation mode setting function 23c sets an irradiation mode, which satisfies a criterion for a chemical reaction of a drug binding to a tumor by a light beam, to the tumor site T1 identified by the identifying function 23b (step S103). For example, the irradiation mode setting function 23c sets, as the irradiation mode, any of an irradiation dose, an irradiation time, and an irradiation intensity in a range including the tumor site T1. The irradiation intensity is an intensity of a light beam emitted from the light beam irradiation device (diffuser), and the irradiation time is a length of time when the light beam is emitted. The irradiation dose is, for example, the product of the irradiation time and the irradiation intensity. For example, the irradiation mode setting function 23c sets, as the irradiation mode, a distribution of the irradiation dose in the range including the tumor site T1.

In one example, the irradiation mode setting function 23c sets an irradiation range group including a combination of an irradiation region R11, an irradiation region R12, an irradiation region R13, and an irradiation region R14 to the tumor site T1, as illustrated in FIG. 3. Specifically, the irradiation mode setting function 23c sets the irradiation range group so that the entire tumor site T1 is covered by at least one irradiation region. Then, the irradiation mode setting function 23c sets any of the irradiation dose, the irradiation time, and the irradiation intensity in the irradiation range group, as the irradiation mode.

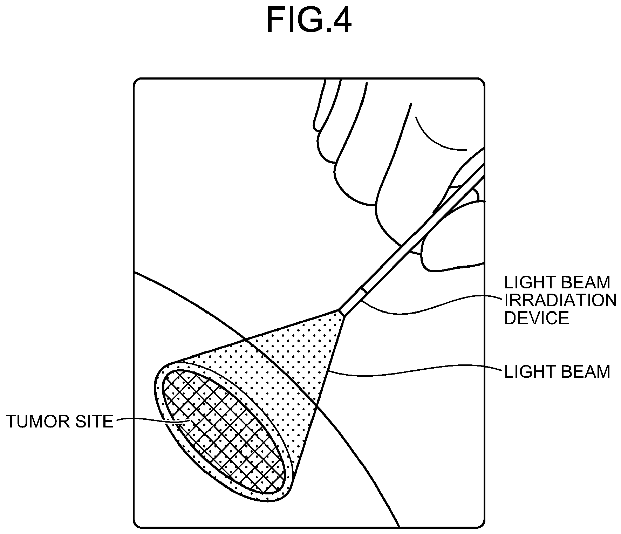

The irradiation range group illustrated in FIG. 3 is set so that individual irradiation ranges have sizes substantially identical to each other, and a part of each irradiation range overlaps with other adjacent irradiation ranges. For example, in a case illustrated in FIG. 4, a light beam emitted from the light beam irradiation device to the tumor site T1 is illuminated within a range with a substantially circular shape. The irradiation mode setting function 23c can set the irradiation range group according to a shape and size of the light beam emitted from the light beam irradiation device. The irradiation ranges included in the irradiation range group may have different sizes. A shape of each of the irradiation ranges included in the irradiation range group may not be circular. For example, in a case in which the shape of each of the irradiation ranges included in the irradiation range group is rectangular, each of the irradiation ranges may not overlap with other adjacent irradiation ranges.

For example, the irradiation mode setting function 23c acquires the irradiation intensity of a light beam emitted from the light beam irradiation device used in the photoimmunotherapy. For example, in a case in which the light beam irradiation device can only emit a light beam with a certain irradiation intensity, the irradiation mode setting function 23c acquires the constant irradiation intensity. In a case in which the light beam irradiation device can adjust the irradiation intensity, the irradiation mode setting function 23c acquires, for example, a predetermined irradiation intensity corresponding to a type of the drug or the tumor site T1. Then, the irradiation mode setting function 23c sets the irradiation time to satisfy the irradiation dose required to chemically react the drug binding to the tumor at the acquired irradiation intensity.

For example, the irradiation mode setting function 23c sets the irradiation time for each of the irradiation regions included in the irradiation region group. That is, the irradiation mode setting function 23c sets a distribution of the irradiation time as the irradiation mode. The irradiation mode setting function 23c may set different irradiation times for the individual irradiation regions or may set the same irradiation time for all of the irradiation regions. Hereinbelow, a case in which "IRRADIATION TIME: 5 MINUTES" is set as the irradiation mode for each of the irradiation regions will be described.

In a case in which a cone beam-shaped light beam is emitted from the light beam irradiation device as illustrated in FIG. 4, a size of the light beam varies depending on a distance from the light beam irradiation device to a body surface of the subject. That is, as the distance from the light beam irradiation device to the body surface of the subject increases, a larger region is irradiated with the light beam. The irradiation mode setting function 23c may set the irradiation region group illustrated in FIG. 3, as well as the distance from the light beam irradiation device to the body surface of the subject.

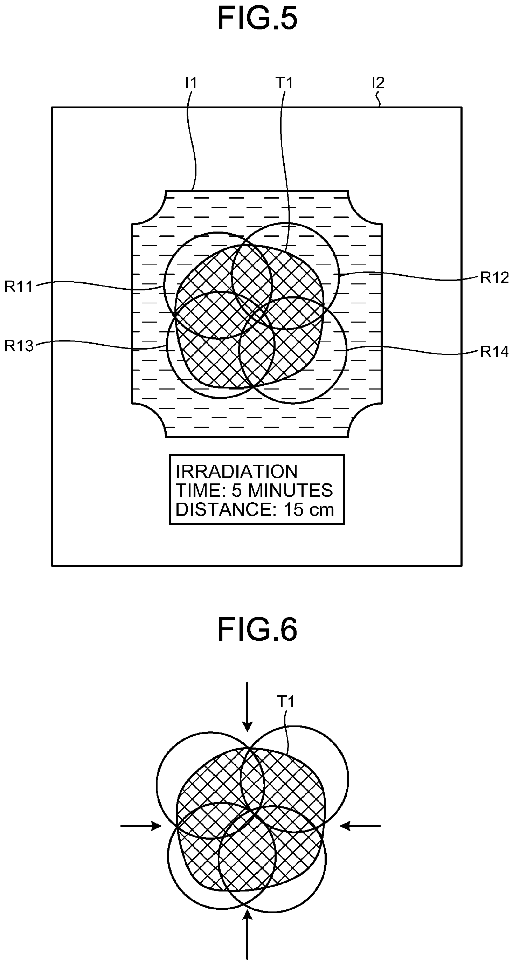

Next, the guidance information generating function 23d generates guidance information for guiding light beam irradiation by the light beam irradiation device based on the irradiation mode set by the irradiation mode setting function 23c (step S104). For example, as illustrated in FIG. 5, the guidance information generating function 23d generates, on an image I1 acquired for the subject, a display image in which the tumor site T1 identified by the identifying function 23b and the irradiation region group set by the irradiation mode setting function 23c are set. Furthermore, the guidance information generating function 23d generates a display area indicating text information such as "IRRADIATION TIME: 5 MINUTES" and "DISTANCE: 15 cm". The guidance information generating function 23d generates a display image I2 including these display image and display area, as the guidance information for guiding light beam irradiation by the light beam irradiation device. The guidance information generated by the guidance information generating function 23d is transmitted to the light beam irradiation supporting apparatus 30 by the control function 23a (step S105) and provided to the user. For example, a provision function 35c described later can provide the guidance information transmitted from the light beam irradiation planning apparatus 20 to the user.

The image II illustrated in FIG. 5 is, for example, an optical image obtained by imaging the range including the tumor site T1 of the subject. The tumor site T1 may not be depicted in the image I1 unless the tumor site T1 is positioned on the body surface. The image I1 may also be an endoscopic image. In the following description, the "body surface" is described as including not only the skin but also any surface that can be reached without surgical procedures such as puncture or incision, such as mucous membranes in the oral cavity, digestive system, and respiratory system.

The display of "IRRADIATION TIME: 5 MINUTES" illustrated in FIG. 5 indicates that each of the irradiation regions included in the irradiation region group is irradiated with the light beam for "5 minutes" to cause the drug binding to the tumor to chemically react, thereby exerting the effect on the tumor. The display of "DISTANCE: 15 cm" illustrated in FIG. 5 indicates that a light beam source of the light beam irradiation device is placed at a distance of "15 cm" from the body surface of the subject. That is, in a case in which light of cone-beam-shape is emitted from the light beam irradiation device as illustrated in FIG. 4, the range irradiated with the light beam increases while the irradiation intensity decreases as the distance from the light beam source increases. Emitting light beam at "DISTANCE: 15 cm" enables an irradiation range with a predetermined size to be irradiated with the light beam with a predetermined irradiation intensity.

In FIG. 5, the case in which the display image I2 is generated as the guidance information indicating the irradiation range including the tumor site and the irradiation time in the irradiation range is described, but various modifications can be adopted for the specific aspect of the display image 12. For example, the guidance information generating function 23d may generate the display image I2 to include the irradiation dose and the irradiation intensity instead of the irradiation time. That is, the guidance information generating function 23d can generate, as the guidance information, at least one of the irradiation range including the tumor site and the irradiation dose, the irradiation time, and the irradiation intensity in the irradiation range.

The guidance information generating function 23d may generate, as the guidance information, a procedure of light beam irradiation by the light beam irradiation device. For example, the guidance information generating function 23d may include, in the guidance information, that the irradiation region R11 is firstly irradiated with the light beam, the irradiation region R12 is secondly irradiated with the light beam, the irradiation region R13 is thirdly irradiated with the light beam, and the irradiation region R14 is fourthly irradiated with the light beam. The user referring to the guidance information in FIG. 5, for example, places the light beam source of the light beam irradiation device at a distance of 15 cm from the subject's body surface, and the irradiation region R11 is irradiated with the light beam for 5 minutes, the irradiation region R12 is irradiated with the light beam for 5 minutes, the irradiation region R13 is irradiated with the light beam for 5 minutes, and the irradiation region R14 is irradiated with the light beam for 5 minutes.

In the photoimmunotherapy, a plurality of the light beam irradiation devices may be used. The irradiation mode setting function 23c may set the number of the light beam irradiation devices used in the photoimmunotherapy as the irradiation mode. In a case in which the number of the light beam irradiation devices is set to "two", the guidance information generating function 23d may cause, for example, the guidance information to include the fact that the irradiation region R11 and the irradiation region R12 are firstly irradiated with the light beam, and the irradiation region R13 and the irradiation region R14 are secondly irradiated with the light beam.

In a case in which the light beam in a circular shape is emitted from the light beam irradiation device and a case in which light beam irradiation is performed at the visual discretion of the user as in the conventional methods, omission in light beam irradiation is likely to occur at arc intersection points as indicated by the arrows in FIG. 6. By contrast, according to the light beam irradiation planning apparatus 20 of the first embodiment, since light beam irradiation can be carried out while referring to the guidance information generated in advance, the omission in irradiation illustrated in FIG. 6 can be avoided.

The irradiation mode setting function 23c can set the irradiation mode in consideration of various other pieces of information in addition to the above description.

For example, in a case in which a distance between the body surface of the subject and the tumor site T1 is large, the light beam is attenuated by normal tissues of the subject, which exists on a light beam irradiation path, and the intensity of the light beam emitted to the tumor site T1 decreases. The irradiation mode setting function 23c may set the irradiation mode in consideration of such a decrease in the intensity of the light beam.

For example, the irradiation mode setting function 23c sets, as the irradiation mode, an irradiation dose according to a depth from the body surface of the subject to the tumor site T1. Specifically, the irradiation mode setting function 23c sets the irradiation mode so that the larger the distance from the body surface to the tumor site T1, the longer the irradiation time or the higher the irradiation intensity.

The irradiation mode setting function 23c may also set, as the irradiation mode, an irradiation direction of the light beam to the tumor site T1. For example, in a case in which a depth from the body surface of the subject to the tumor site T1 varies depending on an irradiation direction, the irradiation mode setting function 23c sets the irradiation direction so that the depth is minimized. In a case in which the light beam irradiation device is inserted into an oral cavity, digestive system, respiratory system, or the like and emits light beam thereto, the irradiation mode setting function 23c sets an irradiation direction in consideration of the ease of reaching the light beam irradiation device.

The irradiation mode setting function 23c may set the irradiation mode based on anatomical information corresponding to at least one of the tumor site T1 or a surrounding area of the tumor site T1, and a criterion for a chemical reaction of a drug binding to a tumor by a light beam. For example, the irradiation mode setting function 23c acquires an irradiation mode (irradiation position, irradiation direction, irradiation caliber, irradiation range, irradiation dose, irradiation time, irradiation intensity, and the like) associated with the types of the drug and the tumor site T1. The irradiation mode setting function 23c also acquires anatomical information such as a size of the tumor site T1, a distance from the body surface of the subject to the tumor site T1, and light beam absorption characteristics of a material (medium) interposingly disposed in an irradiation path. The irradiation mode setting function 23c outputs information for identifying whether the irradiation mode in the range including the tumor site T1 satisfies the criterion for the chemical reaction of the drug binding to the tumor by a light beam, based on the acquired irradiation mode and the anatomical information. Upon receiving this output information, the user adjusts the irradiation mode. As a result, the irradiation mode setting function 23c can set the irradiation mode satisfying the criterion for the chemical reaction of the drug binding to the tumor site T1 by a light beam.

In addition, the irradiation mode setting function 23c may also set the irradiation mode in consideration of movement of the tumor site T1.

For example, the tumor site T1 may move under the influence of heartbeat, respiration, or body motion of the subject. In this case, the irradiation mode setting function 23c may set an irradiation range group to regions where the moving tumor site T1 may be positioned.

For example, the irradiation mode setting function 23c sets an extended tumor site where the tumor site T1 illustrated in FIG. 3 extends. In one example, the irradiation mode setting function 23c enlarges the tumor site T1 at a certain magnification rate while maintaining the center position of the tumor site T1 and sets the extended tumor site. Then, the irradiation mode setting function 23c sets the irradiation range group so that the entire extended tumor site is covered by at least one irradiation region.

The magnification rate in a case of setting the extended tumor site may be set in consideration of a movement magnitude at the tumor site T1. For example, in a case in which the tumor site T1 is a site that periodically moves under the influence of breathing, the irradiation mode setting function 23c can estimate the movement magnitude according to a distance from a lung to the tumor site T1 and can set the extended tumor site with a magnification rate according to the estimated result. That is, the irradiation mode setting function 23c can be set so that the smaller the distance from the lung to the tumor site T1, the larger the extended tumor site.

In a case in which the irradiation range group is set to the extended tumor site instead of the tumor site T1, the light beam emitted to normal tissues other than the tumor site T1 is increased. However, the light beam emitted from the light beam irradiation device to the subject is not invasive to the subject. Therefore, in the case in which the irradiation range group is set to the extended tumor site, although the treatment time may be longer, the entire tumor site T1 can be more reliably irradiated with the light beam without affecting the normal tissues of the subject.

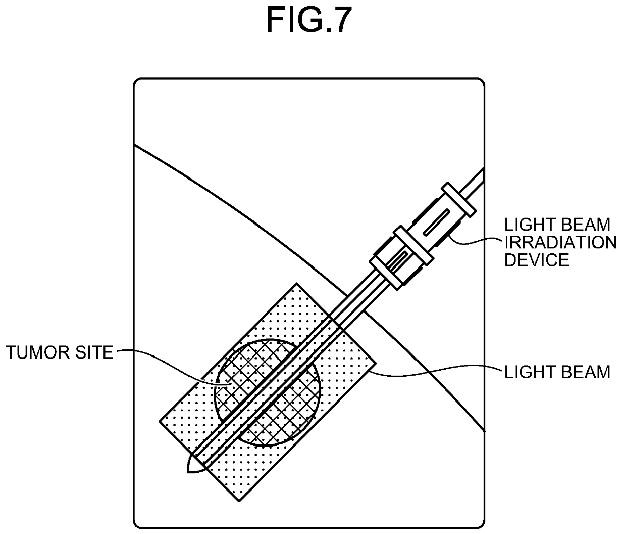

Another method of the photoimmunotherapy in which a puncture is performed by the light beam irradiation device, and the inside of the subject's body is irradiated with the light beam, as illustrated in FIG. 7, has been known. The irradiation mode setting function 23c may set, as the irradiation mode, whether the subject is punctured by the light beam irradiation device.

For example, as photoimmunotherapy for pharyngeal cancer, there are two methods of: emitting a light beam to a body surface (mucosa in the oral cavity); and puncturing the mucosa in the oral cavity by the light beam irradiation device. The irradiation mode setting function 23c can determine whether a puncture is performed, assuming that the puncture is performed in a case in which the distance from the body surface to the tumor site T1 is large. In a case of performing the puncture, the irradiation mode setting function 23c may further generate information indicating a position where the puncture is performed by the light beam irradiation device on the body surface of the subject, as the guidance information. Such information indicating the position can be displayed on, for example, the image I1 illustrated in FIG. 5.

### Second Embodiment

In the first embodiment described above, the light beam irradiation planning apparatus 20 generates the guidance information during the planning, thereby enabling efficient light beam irradiation in the photoimmunotherapy. In a second embodiment, the light beam irradiation supporting apparatus 30 provides irradiation supporting information described later during the execution of the photoimmunotherapy, thereby enabling efficient light beam irradiation in the photoimmunotherapy.

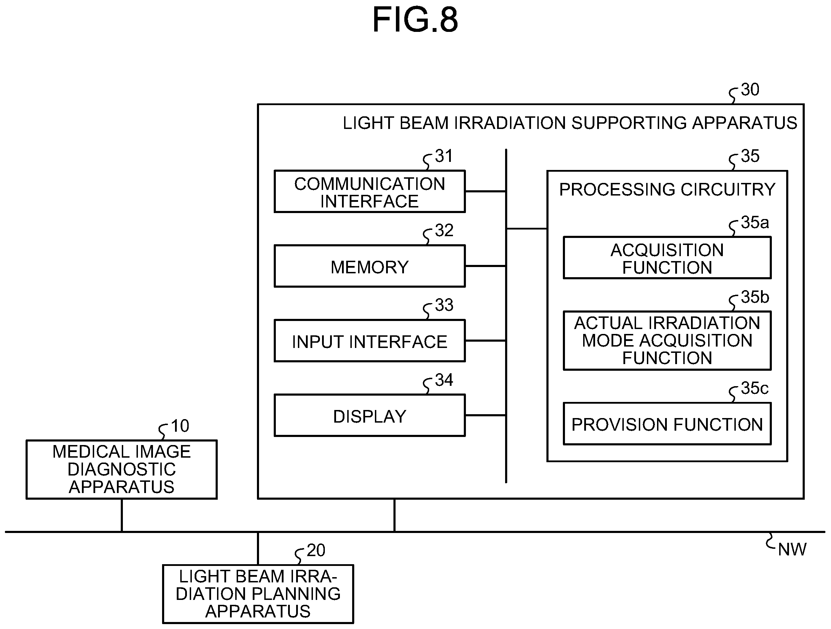

A medical information processing system 1 according to the second embodiment will be described with reference to FIG. 8. Similar to the example illustrated in FIG. 1, the medical information processing system 1 includes a medical image diagnostic apparatus 10, a light beam irradiation planning apparatus 20, and a light beam irradiation supporting apparatus 30. The medical image diagnostic apparatus 10 and the light beam irradiation planning apparatus 20 have the same configurations as those described in the first embodiment.

As illustrated in FIG. 8, the light beam irradiation supporting apparatus 30 is provided with a communication interface 31, a memory 32, an input interface 33, a display 34, and a processing circuitry 35.

The communication interface 31 is composed of, for example, a network card, a network adapter, or the like. The communication interface 31 transmits and receives various pieces of information to and from devices connected via the network NW under the control of the processing circuitry 35.

The memory 32 is composed of, for example, storage devices such as semiconductor memory devices such as RAM, flash memory, and the like, hard disks, and optical disks. The memory 32 stores various pieces of information supplied by the processing circuitry 35. In addition, the memory 32 stores computer programs to be executed by the processing circuitry 35.

The input interface 33 is composed of, for example, a mouse and keyboard, a trackball, a switch, a button, a joystick, a touch pad for input operation by a touch of an operation surface, a touchscreen formed with a display screen and a touch pad, which are integrated, a non-contact input circuit with optical sensors, a voice input circuit, or other interfaces. The input interface 33 receives various input operations from the user and supplies electrical signals corresponding to the received input operations to the processing circuitry 35.

The display 34 is composed of a display device such as a liquid crystal display or a cathode ray tube (CRT) display. The display 34 displays various pieces of information supplied via the processing circuitry 35.

The processing circuitry 35 is composed of an arithmetic processing device such as CPU or MPU. The processing circuitry 35 reads and executes computer programs stored in the memory 32 to function as an acquisition function 35a, an actual irradiation mode acquisition function 35b, and a provision function 35c. The acquisition function 35a is an example of an acquisition unit. The actual irradiation mode acquisition function 35b is an example of an actual irradiation mode acquisition unit. The provision function 35c is an example of a provision unit.

The acquisition function 35a acquires a preset irradiation mode to the tumor site T1. For example, the acquisition function 35a controls the communication interface 31 to acquire the irradiation mode set by the irradiation mode setting function 23c described above. The preset irradiation mode acquired by the acquisition function 35a is hereinafter also referred to as a setting irradiation mode. The actual irradiation mode acquisition function 35b acquires an irradiation mode by which the tumor site T1 is irradiated. That is, the actual irradiation mode acquisition function 35b acquires an irradiation mode for light beam actually emitted by the light beam irradiation device. Hereafter, the irradiation mode for the actually emitted light beam that is acquired by the actual irradiation mode acquisition function 35b is also referred to as an actual irradiation mode. The provision function 35c provides irradiation supporting information for supporting light beam irradiation by the light beam irradiation device based on the setting irradiation mode and the actual irradiation mode. For example, the provision function 35c generates irradiation supporting information and displays the irradiation supporting information on the display 34. The details of processing executed by the acquisition function 35a, the actual irradiation mode acquisition function 35b, and the provision function 35c will be described below.

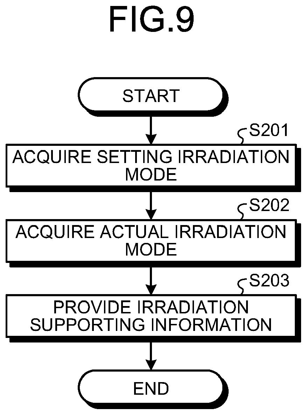

Under such a configuration, the light beam irradiation supporting apparatus 30 in the medical information processing system 1 enables efficient light beam irradiation in the photoimmunotherapy. The details of processing executed by the light beam irradiation supporting apparatus 30 will be described below with a flowchart illustrated in FIG. 9.

First, the acquisition function 35a acquires a setting irradiation mode (step S201). For example, the irradiation mode setting function 23c in the light beam irradiation planning apparatus 20 sets the irradiation mode satisfying the criterion for the chemical reaction of the drug binding to the tumor by a light beam, as described in the first embodiment. The acquisition function 35a acquires, as the setting irradiation mode, the irradiation mode set by the irradiation mode setting function 23c. Alternatively, the acquisition function 35a may also execute the same processing as that of the irradiation mode setting function 23c to set the setting irradiation mode. In this case, the medical information processing system 1 may not include the light beam irradiation planning apparatus 20. Hereinbelow, a case in which "IRRADIATION TIME: 5 MINUTES" is set to each of the irradiation regions included in the irradiation region group illustrated in FIG. 3 as the setting irradiation mode will be described. That is, the acquisition function 35a acquires a distribution of the irradiation time set to the range including the tumor site T1 as the setting irradiation mode.

Next, the actual irradiation mode acquisition function 35b acquires an actual irradiation mode (step S202). For example, the actual irradiation mode acquisition function 35b sequentially acquires the distribution of the irradiation time when the range including the tumor site is actually irradiated while the light beam irradiation is performed by the light beam irradiation device. That is, the actual irradiation mode acquisition function 35b acquires, as the actual irradiation mode, the distribution of the irradiation time when the range including the tumor site is actually irradiated in real time.

For example, while light beam is being emitted by the light beam irradiation device, optical images of the range including the tumor site T1 are sequentially acquired by an optical camera, which is not illustrated in the figure. The optical images may be images imaged by a fixed camera provided in the room or may be images imaged by an endoscope. The actual irradiation mode acquisition function 35b recognizes changes in luminance values and performs color conversion of such optical images to identify a position that is being irradiated with the light beam by the light beam irradiation device within the range including the tumor site T1, and acquire the actual irradiation mode. Specifically, the position irradiated with the light beam by the light beam irradiation device glows and is depicted as a change in color in the optical images. The actual irradiation mode acquisition function 35b can enhance contrast by performing color conversion of the optical images to identify the position that is being irradiated with the light beam by the light beam irradiation device. The actual irradiation mode acquisition function 35b sequentially acquires the optical images and sequentially performs the processing of identifying the position that is being irradiated with the light beam, which enables acquisition of an irradiation time when each position is actually irradiated with the light beam.

A method of identifying the position that is being irradiated the light beam by the light beam irradiation device is not particularly limited. For example, a position and an angle of the light beam irradiation device can be detected by a sensor to identify a position that is being irradiated with a light beam.

Next, the provision function 35c provides irradiation supporting information for supporting light beam irradiation by the light beam irradiation device based on the setting irradiation mode and the actual irradiation mode (step S203). For example, the provision function 35c provides, as the irradiation supporting information, information that enables identification of a relationship between the distribution of the irradiation time included in the setting irradiation mode and the distribution of the irradiation time included in the actual irradiation mode.

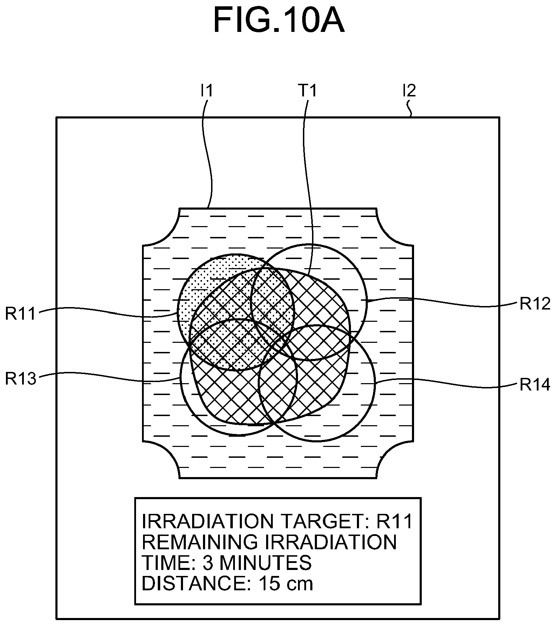

Hereinbelow, examples for providing the irradiation supporting information will be described with reference to FIGS. 10A to 10D. In FIGS. 10A to 10D, "IRRADIATION TIME: 5 MINUTES" is set to each of the irradiation regions R11 to R14 as the setting irradiation mode.

For example, the provision function 35c first causes the display 34 to display a display screen illustrated in FIG. 5 as the irradiation supporting information. That is, the provision function 35c causes the display 34 to display a superimposed image of the image I1 and the distribution of the irradiation time included in the setting irradiation mode as the irradiation supporting information. The image I1 is an optical image sequentially acquired for the range including the tumor site T1. For example, the image I1 is acquired in real time by an endoscope integrated with the light beam irradiation device. Referring to the display screen, the user operates the light beam irradiation device to emit a light beam to each of the irradiation regions R11 to R14 sequentially. For example, the user sets a distance of "15 cm" from the body surface of the subject and operates the light beam irradiation device to sequentially irradiate each of the irradiation region R11, the irradiation region R12, the irradiation region R13, and the irradiation region R14 with the light beam. For example, the user firstly allows the irradiation region R11 to be irradiated with a light beam.

While the irradiation region R11 is irradiated with the light beam, the actual irradiation mode acquisition function 35b sequentially acquires actual irradiation modes. For example, the actual irradiation mode acquisition function 35b executes color recognition processing on the optical images that are sequentially acquired for the range including the tumor site T1 to determine whether the light beam irradiation has been started for the irradiation region R11, and whether the light beam irradiation is continuing for the irradiation region R11, thereby sequentially acquiring a total irradiation time for the irradiation region R11. For example, when the irradiation region R11 has been irradiated with the light beam for "2 MINUTES", the actual irradiation mode acquisition function 35b acquires information of "IRRADIATION TARGET: R11, IRRADIATION TIME: 2 MINUTES" as the actual irradiation mode.

Here, the setting irradiation mode includes information of "IRRADIATION TIME: 5 MINUTES" set to each of the irradiation regions, as the distribution of the irradiation time. In addition, the actual irradiation mode includes the information of "IRRADIATION TARGET: R11, IRRADIATION TIME: 2 MINUTES", as the distribution of the irradiation time. Therefore, the provision function 35c causes the irradiation supporting information to be displayed so that the user can recognize that the irradiation region R11 is required to be irradiated for remaining "3 MINUTES", and that light beam irradiation has not yet been performed on the other irradiation regions. Specifically, as illustrated in FIG. 10A, the provision function 35c can cause information that the irradiation target at this time is the irradiation region R11 and that the remaining irradiation time assigned to the irradiation region R11 is "3 MINUTES" to be displayed by letters.

For example, the provision function 35c provides information that enables identification of a relationship between the distribution of the irradiation time included in the setting irradiation mode and the distribution of the irradiation time included in the actual irradiation mode. Specifically, the provision function 35c provides information that enables identification of the fact that "2 MINUTES" of irradiation has been completed for the irradiation time of "5 MINUTES" set to the irradiation region R11, and that no light beam irradiation has been performed for the irradiation regions other than the irradiation region R11.

For example, as illustrated in FIG. 10A, the provision function 35c causes the irradiation region R11 in the irradiation region group to be displayed with a color corresponding to the irradiation time. For example, the provision function 35c causes the irradiation region R11 to be displayed with a color corresponding to a ratio of the irradiation time "2 MINUTES" included in the actual irradiation mode to the irradiation time "5 MINUTES" included in the setting irradiation mode, or a difference therebetween. For example, the provision function 35c causes the irradiation region R11 to be displayed with a transparency of "100%" when the irradiation time included in the actual irradiation mode is "0 MINUTES", and causes the irradiation region R11 to be displayed so that the transparency gradually decreases as the time when the irradiation region R11 is irradiated with the light beam increases. That is, the provision function 35c provides the irradiation supporting information while updating the irradiation supporting information sequentially according to the actual irradiation mode. In FIGS. 10A to 10D, it is described that the tumor site T1 is also displayed, but the tumor site T1 may not be displayed as appropriate.

More specifically, as illustrated in FIG. 10A, the provision function 35c causes the display 34 to display a superimposed image of the image I1 and each irradiation region colored according to the irradiation time, as the irradiation supporting information. That is, the provision function 35c acquires the relationship between the distribution of the irradiation time included in the setting irradiation mode and the distribution of the irradiation time included in the actual irradiation mode, and causes the display 34 to display a superimposed image of the distribution indicating the relationship and the image I1, as the irradiation supporting information.

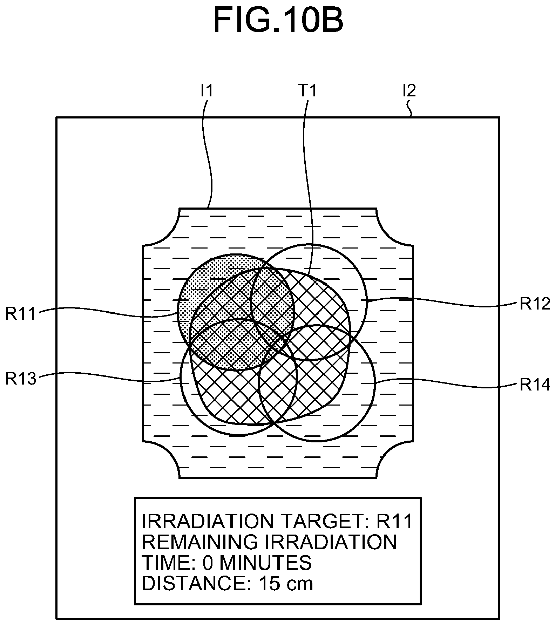

When "5 MINUTES" of the light beam irradiation for the irradiation region R11 is completed, the provision function 35c causes letters of "IRRADIATION TARGET: R11, REMAINING IRRADIATION TIME: 0 MINUTES" as illustrated in FIG. 10B to be displayed. In addition, the provision function 35c reduces the transparency of the irradiation region R11 in the irradiation region group. That is, the provision function 35c causes the irradiation region R11 in the irradiation region group to be displayed with the transparency indicating that irradiation has been completed. In addition, the provision function 35c causes other irradiation regions that are not irradiated with the light beam to be displayed with a transparency of "100%".

After "5 MINUTES" of light beam irradiation for the irradiation region R11 is completed, another irradiation region that is not yet irradiated is started to be irradiated with the light beam. Here, the provision function 35c may provide information indicating the next irradiation region to be irradiated with the light beam in the irradiation region group, as the irradiation supporting information. For example, the provision function 35c provides information indicating the irradiation region R12, the irradiation region R13, and the irradiation region R14 in the irradiation region group, which are not yet irradiated with the light beam, as the irradiation supporting information.

In other words, the provision function 35c may provide information that enables identification of the irradiation range where there is a difference between the irradiation time included in the setting irradiation mode and the irradiation time included in the actual irradiation mode, as the irradiation supporting information. That is, in the irradiation region R11 where "5 MINUTES" of light beam irradiation has been completed, there is no difference between the irradiation time for "5 MINUTES" included in the setting irradiation mode and the irradiation time for "5 MINUTES" included in the actual irradiation mode. On the other hand, the irradiation time included in the actual irradiation mode for the other irradiation ranges excluding the irradiation region R11 is "0 MINUTES" compared with the irradiation time for "5 MINUTES" included in the setting irradiation mode, resulting in a difference of "5 MINUTES". The provision function 35c provides information that enables identification of these other irradiation ranges, as the irradiation supporting information.

Alternatively, the provision function 35c may designate any one of the irradiation regions as the next irradiation region to be irradiated. For example, as the setting irradiation mode, it is preset that irradiation is performed in order of the irradiation region R11, the irradiation region R12, the irradiation region R13, and the irradiation region R14. In this case, after "5 MINUTES" of light beam irradiation is completed on the irradiation region R11, the provision function 35c provides information indicating that the next irradiation region to be irradiated is the irradiation region R12, as the irradiation supporting information. For example, the provision function 35c may cause the next irradiation region to be irradiated is the irradiation region R12 to be displayed by letters, or may indicate that the next irradiation region to be irradiated is the irradiation region R12 by changing a color of the irradiation region R12 in the display illustrated in FIG. 10B.

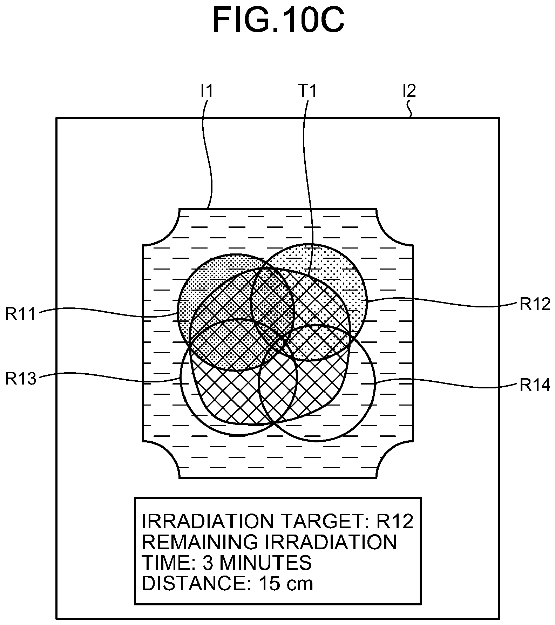

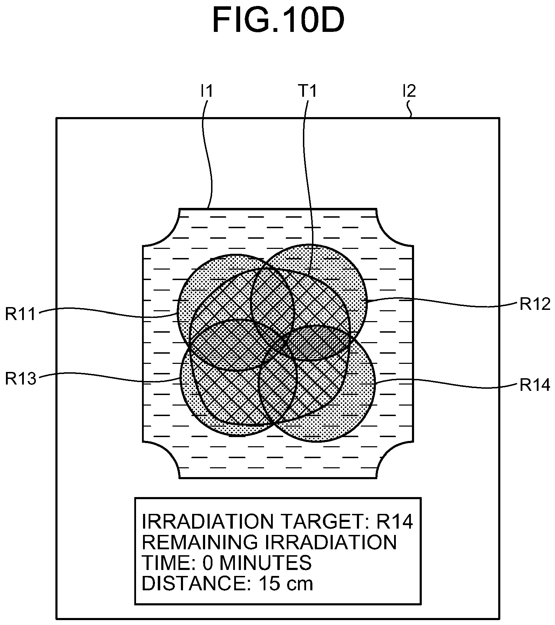

When "2 MINUTES" of the light beam irradiation for the irradiation region R12 is completed, the provision function 35c causes letters of "IRRADIATION TARGET: R12, REMAINING IRRADIATION TIME: 3 MINUTES" as illustrated in FIG. 10C to be displayed as the irradiation supporting information. In addition, the provision function 35c causes the irradiation region R11 to be displayed with the transparency indicating that irradiation has been completed, the irradiation region R12 to be displayed with a transparency according to the irradiation time "2 MINUTES" included in the actual irradiation mode, and other irradiation regions that are not irradiated with the light beam to be displayed with a transparency of "100%", in the irradiation region group. When "5 MINUTES" of the light beam irradiation for each of the irradiation regions R11 to R14 is completed, the provision function 35c causes each of the irradiation regions to be displayed with a transparency indicating that irradiation is completed, as illustrated in FIG. 10D. Therefore, the user can confirm that the photoimmunotherapy procedure has been completed and end the procedure.

With reference to FIGS. 10A to 10D, the examples of providing "REMAINING IRRADIATION TIME" have been described. However, the embodiments are not limited thereto, and the irradiation time included in the setting irradiation mode may be provided along with the irradiation time included in the actual irradiation mode. For example, instead of displaying "REMAINING IRRADIATION TIME: 3 MINUTES" in FIG. 10A, the irradiation time "5 MINUTES" included in the setting irradiation mode and "2 MINUTES" included in the actual irradiation mode may be displayed. That is, the provision function 35c may provide, as the irradiation supporting information, information indicating the setting irradiation mode together with information indicating the actual irradiation mode.

It is also possible to replace the "irradiation time" with the "irradiation dose" in each of the above-described embodiments. For example, in FIGS. 10A to 10D, "REMAINING IRRADIATION DOSE" can be displayed instead of "REMAINING IRRADIATION TIME". In addition, the distribution of the irradiation dose can be used instead of the irradiation time distribution described above. Specifically, the irradiation time can be converted into the irradiation dose by multiplying the irradiation intensity. Usually, the irradiation intensity is a known value controlled by the light beam irradiation device. In FIG. 1, the light beam irradiation planning apparatus 20 and the light beam irradiation supporting apparatus 30 are illustrated as separate devices, but the devices may be configured as a single device.

In each of the above-described embodiments, the light beam emitted from the light beam irradiation device is typically, but not limited to, near-infrared light beam. That is, a wavelength of the light beam emitted from the light beam irradiation device can be changed as needed.

The term "processor" used in the above description refers to, for example, circuits such as CPU, graphics processing unit (GPU), application specific integrated circuit (ASIC), programmable logic device (for example, simple programmable logic device (SPLD), complex programmable logic device (CPLD), and field programmable gate array (FPGA)). In a case in which the processor is CPU, for example, the processor reads and executes a computer program stored in a memory circuit to implement a function. On the other hand, in a case in which the processor is, for example, ASIC, the function is incorporated directly as a logic circuit within a circuit of the processor, instead of storing the computer program in a storage circuit. Each of the processors in the embodiments is not limited to a case where each processor is configured as a single circuit, but may also be configured as a single processor by combining multiple independent circuits to implement functions thereof. Furthermore, a plurality of components in each of the figures may be integrated into a single processor to implement the functions thereof.

In FIG. 1, it is described that the single memory 22 stores a computer program corresponding to each processing function of the processing circuitry 23. In FIG. 8, it is described that the single memory 32 stores a computer program corresponding to each processing function of the processing circuitry 35. However, the embodiments are not limited thereto. For example, a plurality of the memories 22 can be distributed and arranged, and the processing circuitry 23 can be configured to read the corresponding computer programs from the individual memories 22. Similarly, a plurality of the memories 32 can be distributed and arranged, and the processing circuitry 35 can be configured to read the corresponding computer programs from the individual memories 32. Instead of storing the computer programs in the memory 22 or the memory 32, the computer programs can be configured to be incorporated directly into the circuit of the processor. In this case, the processor reads and executes the computer programs stored in the circuit to implement the functions.

Each component of each device according to the above-described embodiments is a functional concept and is not necessary to be physically configured as illustrated in the figures. That is, the specific form of dispersion or integration of individual devices is not limited to those illustrated in the figures, and all or some of the devices can be configured to be functionally or physically dispersed or integrated by any unit according to various loads or usage conditions. Furthermore, all or some of the individual processing functions performed by the individual devices can be implemented by CPU and computer programs analyzed and executed by the CPU, or by hardware using wired logic.

The medical information processing method described in the embodiments above can be implemented by executing pre-prepared computer programs on a computer such as a personal computer or workstation. These computer programs can be distributed via the Internet or other networks. The computer program can also be recorded on a non-transitory recording medium readable by a computer, such as a hard disk, flexible disk (FD), CD-ROM, MO, or DVD, and executed by being read from a recording medium by a computer.

According to at least one of the embodiments described above, efficient light beam irradiation is possible in the photoimmunotherapy.

Further embodiments are set out in the following clauses:
1. A light beam irradiation planning apparatus (20) comprising:
   an identifying unit (23b) configured to identify a tumor site included in a medical image;
   an irradiation mode setting unit (23c) configured to set an irradiation mode to the tumor site, the irradiation mode satisfying a criterion for a chemical reaction of a drug binding to a tumor by a light beam; and
   a guidance information generating unit (23d) configured to generate guidance information for guiding light beam irradiation by a light beam irradiation device based on the set irradiation mode.
2. The light beam irradiation planning apparatus (20) according to clause 1, wherein the irradiation mode setting unit (23c) sets, as the irradiation mode, any of an irradiation dose, an irradiation time, and an irradiation intensity in a range including the tumor site.
3. The light beam irradiation planning apparatus (20) according to clause 1, wherein the irradiation mode setting unit (23c) sets, as the irradiation mode, any of an irradiation dose, an irradiation time, and an irradiation intensity in an irradiation range group including a combination of a plurality of irradiation ranges.
4. The light beam irradiation planning apparatus (20) according to clause 3, wherein the irradiation mode setting unit (23c) sets the irradiation range group so that a part of each irradiation range overlaps with other irradiation ranges adjacent to the irradiation range.
5. The light beam irradiation planning apparatus (20) according to clause 3, wherein the irradiation mode setting unit (23c) sets the irradiation range group in which sizes of the irradiation ranges are substantially identical to each other.
6. The light beam irradiation planning apparatus (20) according to clause 1, wherein the irradiation mode setting unit (23c) sets the irradiation mode based on anatomical information corresponding to at least one of the tumor site or a surrounding area of the tumor site, and the criterion.
7. The light beam irradiation planning apparatus (20) according to clause 6, wherein the irradiation mode setting unit **(23c)** outputs information for identifying whether the irradiation mode in the range including the tumor site satisfies the criterion, based on an irradiation condition of the light beam irradiation device and the anatomical information.
8. The light beam irradiation planning apparatus (20) according to clause 1, wherein the guidance information generating unit **(23d)** generates, as the guidance information, a procedure of light beam irradiation by the light beam irradiation device.
9. The light beam irradiation planning apparatus (20) according to clause 1, wherein the guidance information generating unit **(23d)** generates, as the guidance information, at least one of an irradiation range including the tumor site and an irradiation dose, an irradiation time, and an irradiation intensity in the irradiation range.
10. A light beam irradiation supporting apparatus (30) comprising:
   an acquisition unit (35a) configured to acquire a preset setting irradiation mode to a tumor site included in a medical image;
   an actual irradiation mode acquisition unit (35b) configured to acquire an actual irradiation mode indicating that the tumor site is actually irradiated; and
   a provision unit (35c) configured to provide irradiation supporting information for supporting light beam irradiation by a light beam irradiation device based on the setting irradiation mode and the actual irradiation mode.
11. The light beam irradiation supporting apparatus (30) according to clause 10, wherein
   the setting irradiation mode includes a distribution of an irradiation dose or an irradiation time set to a range including the tumor site,
   the actual irradiation mode includes a distribution of an irradiation dose or an irradiation time, which is obtained by the range being actually irradiated, and
   the irradiation supporting information is information that enables identification of a relationship between the distribution of the irradiation dose or the irradiation time included in the setting irradiation mode and the distribution of the irradiation dose or the irradiation time included in the actual irradiation mode.
12. A medical information processing system (1) comprising:
   an identifying unit (23b) configured to identify a tumor site included in a medical image;
   an irradiation mode setting unit (23c) configured to set an irradiation mode to the tumor site, the irradiation mode satisfying a criterion for a chemical reaction of a drug binding to a tumor by a light beam;
   a guidance information generating unit (23d) configured to generate guidance information for guiding light beam irradiation by a light beam irradiation device based on the set irradiation mode; and
   a provision unit (35c) configured to provide the guidance information.
13. A medical information processing system (1) comprising:
   an identifying unit (23b)configured to identify a tumor site included in a medical image;
   an irradiation mode setting unit (23c) configured to set a setting irradiation mode to the tumor site, the setting irradiation mode satisfying a criterion for a chemical reaction of a drug binding to a tumor by a light beam;
   an acquisition unit (35a) configured to acquire the setting irradiation mode;
   an actual irradiation mode acquisition unit (35b) configured to acquire an actual irradiation mode indicating that the tumor site is actually irradiated; and
   a provision unit (35c) configured to provide irradiation supporting information for supporting light beam irradiation by a light beam irradiation device based on the setting irradiation mode and the actual irradiation mode.
14. A medical information processing method comprising:
   identifying a tumor site included in a medical image;
   setting an irradiation mode to the tumor site, the irradiation mode satisfying a criterion for a chemical reaction of a drug binding to a tumor by a light beam; and
   generating guidance information for guiding light beam irradiation by a light beam irradiation device based on the set irradiation mode.
15. A medical information processing method comprising:
   acquiring a preset setting irradiation mode to a tumor site included in a medical image;
   acquiring an actual irradiation mode indicating that the tumor site is actually irradiated; and
   providing irradiation supporting information for supporting light beam irradiation by a light beam irradiation device based on the setting irradiation mode and the actual irradiation mode.

While certain embodiments have been described, these embodiments have been presented by way of example only, and are not intended to limit the scope of the invention as claimed. Indeed, the novel devices, methods and systems described herein may be embodied in a variety of other forms; furthermore, various omissions, substitutions, changes and combinations in the form of the devices, methods and systems described herein may be made without departing from the scope of the invention as claimed. The accompanying claims and their equivalents are intended to cover such forms or modifications as would fall within the scope of the invention as claimed.

## Claims

1. A light beam irradiation planning apparatus (20) comprising:
an identifying unit **(23b)** configured to identify a tumor site included in a medical image;
an irradiation mode setting unit **(23c)** configured to set an irradiation mode to the tumor site, the irradiation mode satisfying a criterion for a chemical reaction of a drug binding to a tumor by a light beam; and
a guidance information generating unit **(23d)** configured to generate guidance information for guiding light beam irradiation by a light beam irradiation device based on the set irradiation mode.

2. The light beam irradiation planning apparatus (20) according to claim 1, wherein the irradiation mode setting unit **(23c)** sets, as the irradiation mode, any of an irradiation dose, an irradiation time, and an irradiation intensity in a range including the tumor site.

3. The light beam irradiation planning apparatus (20) according to claim 1, wherein the irradiation mode setting unit **(23c)** sets, as the irradiation mode, any of an irradiation dose, an irradiation time, and an irradiation intensity in an irradiation range group including a combination of a plurality of irradiation ranges.

4. The light beam irradiation planning apparatus (20) according to claim 3, wherein the irradiation mode setting unit (23c) sets the irradiation range group so that a part of each irradiation range overlaps with other irradiation ranges adjacent to the irradiation range.

5. The light beam irradiation planning apparatus (20) according to claim 3, wherein the irradiation mode setting unit **(23c)** sets the irradiation range group in which sizes of the irradiation ranges are substantially identical to each other.

6. The light beam irradiation planning apparatus (20) according to claim 1, wherein the irradiation mode setting unit **(23c)** sets the irradiation mode based on anatomical information corresponding to at least one of the tumor site or a surrounding area of the tumor site, and the criterion.

7. The light beam irradiation planning apparatus (20) according to claim 6, wherein the irradiation mode setting unit **(23c)** outputs information for identifying whether the irradiation mode in the range including the tumor site satisfies the criterion, based on an irradiation condition of the light beam irradiation device and the anatomical information.

8. The light beam irradiation planning apparatus (20) according to claim 1, wherein the guidance information generating unit **(23d)** generates, as the guidance information, a procedure of light beam irradiation by the light beam irradiation device, or wherein the guidance information generating unit **(23d)** generates, as the guidance information, at least one of an irradiation range including the tumor site and an irradiation dose, an irradiation time, and an irradiation intensity in the irradiation range.

9. A medical information processing system (1) comprising:
the light beam irradiation planning apparatus (20) according to any preceding claim; and
a light beam irradiation supporting apparatus (30) comprising:
an acquisition unit (35a) configured to acquire the irradiation mode as a preset setting irradiation mode;
an actual irradiation mode acquisition unit (35b) configured to acquire an actual irradiation mode indicating that the tumor site is actually irradiated; and
a provision unit (35c) configured to provide irradiation supporting information for supporting light beam irradiation by a light beam irradiation device based on the setting irradiation mode and the actual irradiation mode.

10. A light beam irradiation supporting apparatus (30) comprising:
an acquisition unit (35a) configured to acquire a preset setting irradiation mode to a tumor site included in a medical image;
an actual irradiation mode acquisition unit (35b) configured to acquire an actual irradiation mode indicating that the tumor site is actually irradiated; and
a provision unit (35c) configured to provide irradiation supporting information for supporting light beam irradiation by a light beam irradiation device based on the setting irradiation mode and the actual irradiation mode.

11. The medical information processing system (1) according to claim 9, wherein
the setting irradiation mode includes a distribution of an irradiation dose or an irradiation time set to a range including the tumor site,
the actual irradiation mode includes a distribution of an irradiation dose or an irradiation time, which is obtained by the range being actually irradiated, and
the irradiation supporting information is information that enables identification of a relationship between the distribution of the irradiation dose or the irradiation time included in the setting irradiation mode and the distribution of the irradiation dose or the irradiation time included in the actual irradiation mode.

12. A medical information processing system (1) comprising:
the light beam irradiation planning apparatus (20) according to any of claims 1 to 8; and
a provision unit (35c) configured to provide the guidance information.

13. A medical information processing system (1) comprising:
the light beam irradiation supporting apparatus (30)according to claim 10;
an identifying unit (23b) configured to identify a tumor site included in a medical image; and
an irradiation mode setting unit (23c) configured to set the setting irradiation mode to the tumor site, the setting irradiation mode satisfying a criterion for a chemical reaction of a drug binding to a tumor by a light beam.

14. A medical information processing method comprising:
identifying a tumor site included in a medical image;
setting an irradiation mode to the tumor site, the irradiation mode satisfying a criterion for a chemical reaction of a drug binding to a tumor by a light beam; and
generating guidance information for guiding light beam irradiation by a light beam irradiation device based on the set irradiation mode.

15. A medical information processing method comprising:
acquiring a preset setting irradiation mode to a tumor site included in a medical image;
acquiring an actual irradiation mode indicating that the tumor site is actually irradiated; and
providing irradiation supporting information for supporting light beam irradiation by a light beam irradiation device based on the setting irradiation mode and the actual irradiation mode.
